(19) Europäisches Patentamt
European Patent Office
Office européen des brevets

(11) Publication number: **0 036 638**
**A2**

(12) **EUROPEAN PATENT APPLICATION**

(21) Application number: **81102071.8**

(22) Date of filing: **19.03.81**

(51) Int. Cl.³: **A 01 N 43/40**, C 07 D 213/89 // C07D213/70, C07D213/61

(30) Priority: **24.03.80 US 133256**

(43) Date of publication of application: **30.09.81 Bulletin 81/39**

(84) Designated Contracting States: **AT BE CH DE FR GB IT LI LU NL SE**

(71) Applicant: **DIAMOND SHAMROCK CORPORATION, 1100 Superior Avenue, Cleveland Ohio 44114 (US)**

(72) Inventor: **Brand, William W., 6223 Indian Point Road, Painesville Ohio 44077 (US)**
Inventor: **Gullo, James M., 3259 South Ridge Road, Perry Ohio 44081 (US)**

(74) Representative: **Weickmann, Heinrich, Dipl.-Ing Patentanwälte Dipl.Ing.H.Weickmann, Dipl.Phys.Dr.K.Fincke Dipl.Ing.F.A.Weickmann Dipl.Chem.B.Huber, Dr.-Ing.H.Liska Möhlstrasse 22, D-8000 München 86 (DE)**

(54) **Herbicidal and plant growth regulant 2-sulfinyl and 2-sulfonyl pyridine N-oxides.**

(57) Compounds of the general formula:

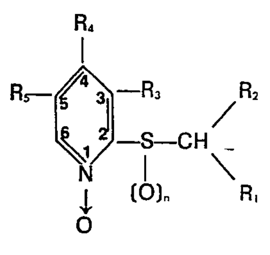

I

are useful in controlling, inhibiting or modifying the growth of plants.

EP 0 036 638 A2

# HERBICIDAL AND PLANT GROWTH REGULANT 2-SULFINYL AND 2-SULFONYL PYRIDINE N-OXIDES

## BACKGROUND OF THE INVENTION

The present invention relates generally to substituted pyridine N-oxide sulfoxides and sulfones which possess valuable herbicidal and other plant growth regulant properties. The instant invention further relates to active plant regulant compositions and to methods for controlling, inhibiting or modifying the growth of vegetation.

Various unsubstituted 2-thio-, 2-sulfinyl-, or 2-sulfonyl-pyridine N-oxide derivatives of the general formula:

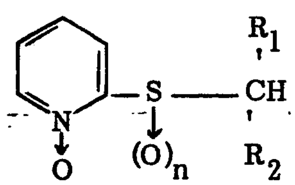

unsubstituted in the 3- through 6-positions of the pyridine ring are disclosed in U.S. Patent Nos. 3,960,542, June 1, 1976; 4,019,893, April 26, 1977; and 4,050,921, September 27, 1977, as herbicidal and plant growth regulant agents.

U.S. Patent Nos. 3,155,671, 3,107,994, 3,535,328, 3,772,307 and 3,984,560 are representative of the state of the art with respect to other pyridine N-oxides.

## SUMMARY OF THE INVENTION

It is, therefore, a primary object of the present invention to afford novel ring substituted 2-sulfinyl and 2-sulfonyl pyridine N-oxide compounds.

It is a further object of the present invention to provide compositions containing the active pyridine N-oxide sulfoxide and sulfone derivatives of the present invention suitably formulated for direct and indirect application to plants and plant environs to obtain desired plant growth regulant effects.

These and other similar objects, advantages and features are accomplished according to the methods, compositions and products of the present invention comprised of novel substituted pyridine N-oxide derivatives, compositions and formulations thereof and plant growth regulant methods employing same.

## DESCRIPTION OF THE PREFERRED EMBODIMENTS

It has now been found, in accordance with the present invention, that substituted pyridine N-oxide compounds of the general formula:

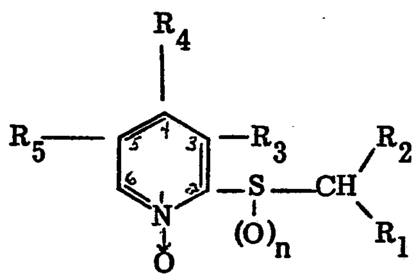

I

wherein n is 1 or 2;

$R_1$ is hydrogen or methyl;

$R_2$ is phenyl, biphenyl, naphthyl or phenyl substituted with at least one substituent selected from the group consisting of halogen, $C_1$-$C_4$ alkyl, trifluoromethyl, cyano or nitro;

$R_3$-$R_5$ are the same or different and selected from the group consisting of hydrogen, halogen, $C_1$-$C_4$ alkyl, phenyl or cyano;

subject to the proviso that at least one of $R_3$-$R_5$ is other than hydrogen are useful in controlling, inhibiting or modifying the growth of plants.

More particularly, the present invention contemplates methods of selectively controlling or combating undesired plant systems, e.g., broadleaf weeds and grasses, especially preemergence control of grasses, comprising applying to the plant locus or situs, a herbicidally effective amount of an active compound of the invention alone or in combination with a preselected carrier,

vehicle or adjuvant. The compounds of the invention also evidence valuable plant growth regulant properties. As used herein, the expression "plant growth regulant" or "plant regulator" is defined as a substance which accelerates, retards or otherwise alters the natural growth, development or maturation of plants. Such observable plant growth regulant effects, in general, include turf growth retardation, e.g., stem and leaf length inhibition, root growth promotion; delayed senescence; yield enhancement; nonphytotoxic herbicide-like responses and the like. The compounds of the present invention are especially preferred for use as plant growth regulants on ornamental turfgrasses to inhibit top growth while maintaining or stimulating root growth. It will be understood by those skilled in the art that whether a plant destruction/herbicidal action or nonphytotoxic plant growth regulant effect is to be obtained by application of the compounds of the invention will vary, not only with the specific compound employed, but also with the relative amount or concentration of the specific active ingredient, the particular plant species being treated, the developmental stage of the plant, the particular formulation or mode of application employed, e.g., seed treatment, soil application, direct plant treatment, etc., as well as soil composition, weather conditions and the like. Generally, the herbicidally active compounds of the present invention will be applied in an amount sufficient to be phytotoxic to undesired vegetation whereas less than herbicidal amounts will be applied to promote nonphytotoxic plant growth regulant responses. Thus, for preemergence herbicidal applications, the herbicidally active pyridine N-oxides of the invention generally will be applied at between about 0.06 kg/hectare to 8.00 kg/hectare; preferably, 0.25 kg/hectare to 4.00 kg/hectare and, most preferably, 0.50 kg/hectare to 2.00 kg/hectare. The desired plant growth regulant effects are obtained at application rates of between about .002 kg/hectare to 8 kg/hectare; preferably, 0.I25 kg/hectare to 2 kg/hectare and, most preferably, 0.125 kg/hectare to 0.5 kg/hectare.

As specifically preferred compounds of the aforementioned general formula for use as preemergence herbicides, there may be especially mentioned 2-(2',5'-dimethylbenzylsulfonyl)-5-bromopyridine N-oxide, 2-(2',5'-dimethylbenzylsulfonyl)-4-methylpyridine N-oxide, 2-(2',5'-dimethylbenzylsulfonyl)-4-ethylpyridine N-oxide, 2-(2',5'-dimethylbenzylsulfonyl)-4-t-butylpyridine N-oxide, 2-(2',5'-dimethylbenzylsulfonyl)-4-chloropyridine N-oxide, 2-(2'-methylbenzylsulfonyl)-4-methylpyridine N-oxide, 2-(2',6'-dichlorobenzylsulfonyl)-4-methylpyridine N-oxide, 2-(2',4',6'-trimethylbenzylsulfonyl)-4-methylpyridine N-oxide,

2-(2',6'-dichlorobenzylsulfonyl)-5-bromopyridine N-oxide, 2-(α ,2',5'-trimethylbenzylsulfonyl)-4-methylpyridine N-oxide and 2-( α-methyl-2',6'-dichlorobenzylsulfonyl)-3-methylpyridine N-oxide.

As representative preferred plant growth regulant compounds, there may be mentioned 2-(2',5'-dimethylbenzylsulfinyl)-4-methylpyridine N-oxide, 2-(2',5'-dimethylbenzylsulfonyl)-4-methylpyridine N-oxide, 2-(2',5'-dimethylbenzylsulfonyl)-5-bromopyridine N-oxide, 2-(2',5'-dimethylbenzylsulfonyl)-4-ethylpyridine N-oxide, 2-(2',5'-dimethylbenzylsulfonyl)-4-t-butylpyridine N-oxide, 2-(2',5'-dimethylbenzylsulfonyl)-4,5-dimethylpyridine N-oxide, 2-(2',5'-dimethylbenzylsulfonyl)-3,4-dimethylpyridine N-oxide, 2-(2'-methylbenzylsulfonyl)-4-methylpyridine N-oxide, 2-(4'-chlorobenzylsulfonyl)-4-methylpyridine N-oxide, 2-(4'-fluorobenzylsulfonyl)-4-methylpyridine N-oxide, 2-(3'-trifluoromethylbenzylsulfonyl)-4-methylpyridine N-oxide, 2-(2',6'-dichlorobenzylsulfonyl)-4-methylpyridine N-oxide, 2-(2',5'-dimethylbenzylsulfinyl)-3-methylpyridine N-oxide, 2-(2',5'-dimethylbenzylsulfonyl)-3-methylpyridine N-oxide, 2-( α ,2',5'-trimethylbenzylsulfonyl)-4-methylpyridine N-oxide, and 2-benzylsulfonyl-4-methylpyridine N-oxide.

The substituted pyridine N-oxide sulfoxides and sulfones of the present invention may be prepared by a number of alternative acceptable methods.

One preferred method for the preparation of the 2-sulfonyl compounds of the invention involves the condensation of an appropriately substituted 2-halopyridine, and an appropriately substituted aralkyl, e.g., benzyl, mercaptan in the presence of a base to form a pyridyl sulfide. The pyridyl sulfide can then be oxidized with at least three equivalents of a known oxidizing agent, e.g., hydrogen peroxide, in a suitable solvent, such as water or acetic acid, to give the desired pyridine N-oxide sulfone as depicted in the following general reaction scheme.

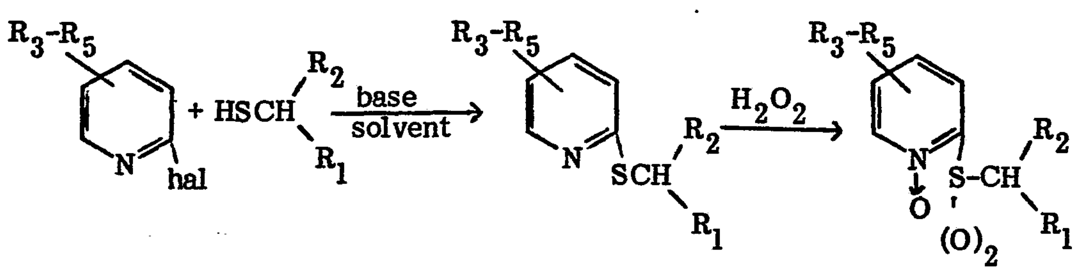

Alternatively, a substituted 2-halopyridine N-oxide can be used in place of the substituted 2-halopyridine to give a pyridine N-oxide sulfide. Only two equivalents of an oxidizing agent such as hydrogen peroxide or m-chloroperbenzoic acid are then required to give the product pyridine N-oxide sulfone.

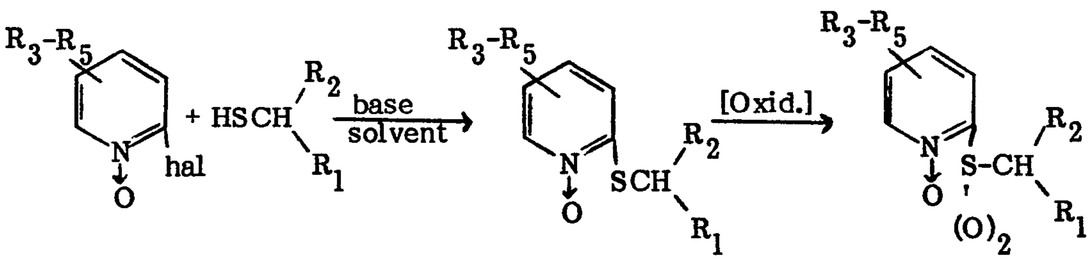


Another preferred method for the preparation of the desired pyridine N-oxide sulfones involves the condensation of a substituted 2-mercaptopyridine with a substituted aralkyl halide to give the intermediate pyridyl sulfides, which are then oxidized to the pyridine N-oxide sulfones in the same manner as described above.

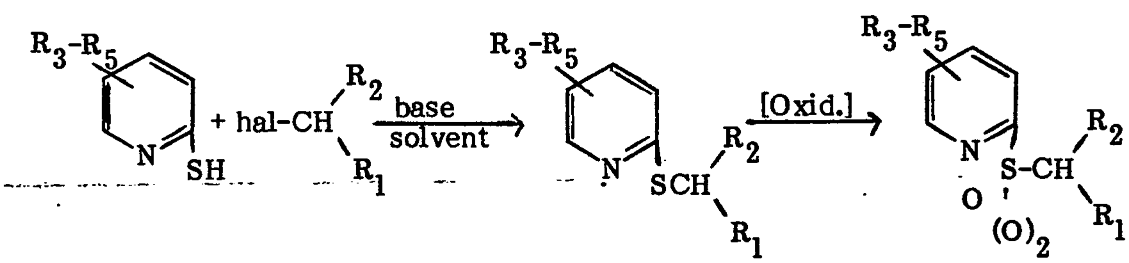


Likewise, substituted 2-mercaptopyridine N-oxides can be condensed with substituted aralkyl halides, and the resulting pyridine N-oxide sulfides can be oxidized to the pyridine N-oxide sulfones with two equivalents of an oxidizing agent according to the following reaction:

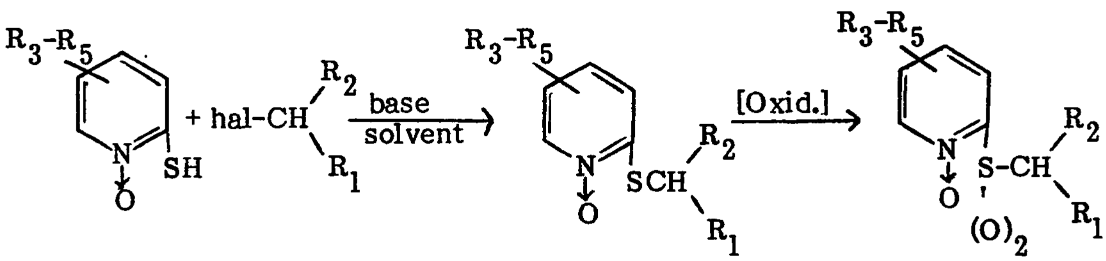

Still another method of making pyridine N-oxide sulfones involves a modification of the procedure described by Abramovitch for preparing 1-hydroxy-2-pyridinethiones [R. A. Abramovitch and E. E. Knaus, J. Heterocyclic Chem., 12, 683 (1975)] in which an appropriately substituted pyridine N-oxide is deprotonated in the 2-position with n-butyllithium at low temperature, followed by thiation with elemental sulfur, and then benzylation with the appropriately substituted benzyl halide. The product isolated is the corresponding substituted pyridine N-oxide sulfide which can then be oxidized to the sulfone as described previously. If the pyridine N-oxide is not symmetrically substituted, this method may give a mixture of isomers which can then be separated by conventional methods such as recrystallization, distillation, chromatography, etc.

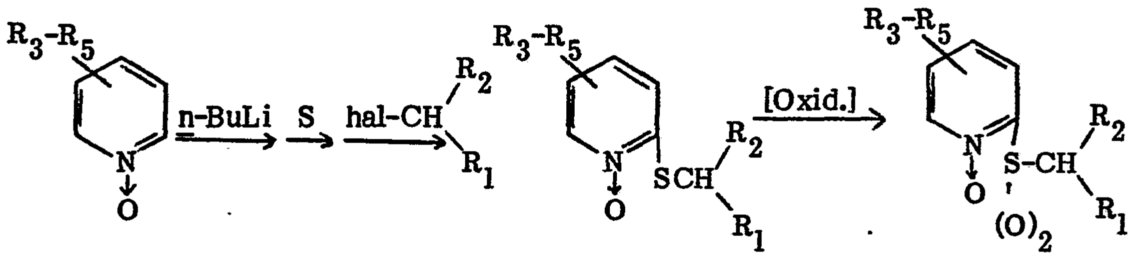


Halo substituted pyridine N-oxide sulfones can be prepared through intermediates according to the following reaction scheme employing strong oxidation conditions (for instance, anhydrous trifluoroperacetic acid):

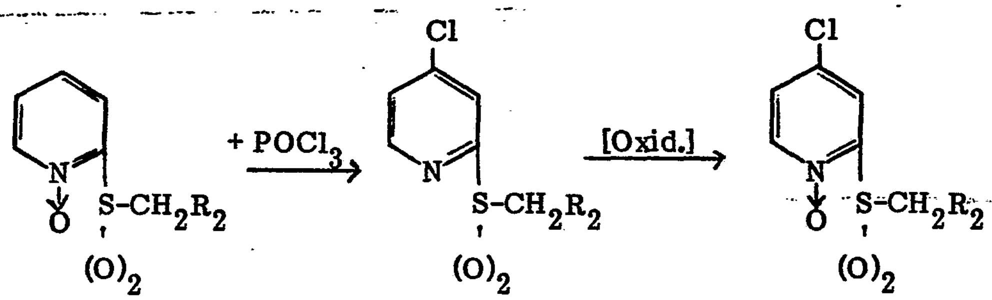


The pyridine N-oxide sulfoxides of the invention are prepared by methods directly analogous to those used to prepare the pyridine N-oxide sulfones, except that only one equivalent of the oxidizing agent (e.g., m-chloroperbenzoic acid) is used to oxidize the corresponding pyridine N-oxide sulfides. This method requires that the oxygen already be present on the pyridine nitrogen before oxidation of the sulfur, i.e., a pyridine N-oxide sulfide intermediate is required.

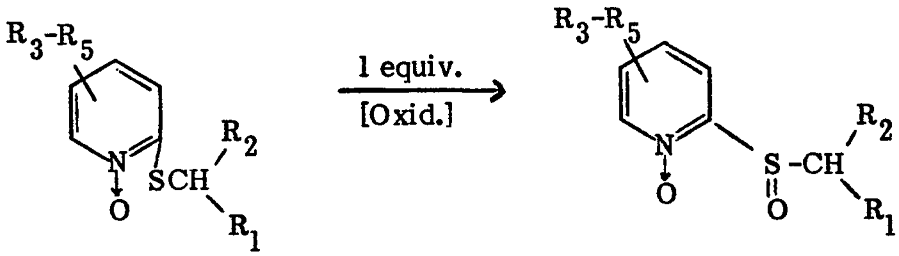


The benzyl and pyridyl mercaptans used in the foregoing reactions can be purchased or can be readily prepared by generally known methods of preparing mercaptans such as reaction of a benzyl or pyridyl halide with thiourea, followed by hydrolysis of the resulting thiouronium salt.

The benzyl halides can be purchased or prepared from the corresponding alcohols and $PBr_3$ or HBr. The pyridyl halides can be purchased or prepared by generally known methods such as diazotization of an aminopyridine or reaction of a pyridine N-oxide and $POCl_3$.

The active compounds according to the instant invention may be utilized, if desired or necessary, in the form of acceptable formulations or compositions containing liquid or solid inert pesticidal carriers or adjuvants in addition to the active agent(s) to provide solutions, emulsions, suspensions, dusts, wettable powders, etc., which facilitate the use and application of the compounds of the invention to preselected substrates. The aforesaid compositions and formulations may be prepared in a known manner, for instance, by admixing the active compounds of the invention with dispersible liquid diluents and carriers optionally in combination with other vehicles, such as surface-active agents, including emulsifying agents or dispersing agents and suitable solubilizing or diluting solvents as described in the above-mentioned U.S. Patent Nos. 3,960,542 or 4,050,921. As suitable carriers or vehicles, there may be mentioned aromatic hydrocarbons (e.g., benzene, toluene, xylene, etc.), halogenated aromatic hydrocarbons (e.g., chlorobenzenes), paraffins (e.g., petroleum fractions), chlorinated aliphatic hydrocarbons (e.g., methylene chloride, etc.), alcohols (e.g., methanol, ethanol, propanol, butanol, etc.), ethers and ether alcohols (e.g., glycol monomethyl ether, etc.), amines (e.g., ethanolamine, etc.), amides (e.g., dimethylformamide, etc.), ketones (e.g., acetone, etc.) and water as well as other conventional solvents. Moreover, solid inert carriers such as kaolins, alumina, silica, calcium carbonate, talc or kieselguhr may be employed. Appropriate nonionic and anionic emulsifying agents or surface active

agents, such as polyethylene oxide esters of fatty acids and fatty alcohols, alkyl sulfonates and aryl sulfonates may be used in conjunction with the aforesaid carriers, vehicles, adjuvants and solvents.

The compositions in accordance with the present invention may also contain other compatible growth regulants, fungicides, nematocides, insecticides, fertilizers and other known herbicides. According to the accepted practices in the formulating art, when combined with other active agents or with carriers, vehicles and the like, the compositions of the invention contain from about 0.01 percent to about 99 percent, by weight, of the instant compounds as the active components thereof.

The active compounds and formulated compositions of the invention may be applied, for example, by spraying, atomizing, dusting, soil injection, seed pretreatment, etc., to obtain the desired preemergence herbicidal and plant growth regulant responses.

The following nonlimiting examples are afforded in order that those skilled in the art may more readily understand the present invention and specific preferred embodiments thereof.

## Example 1

## 2-(2',5'-Dimethylbenzylthio)-4-methylpyridine

A mixture of 17.2 g (0.1 mol) of 2-bromo-4-picoline, 15.2 g (0.1 mol) of 2,5-dimethylbenzyl mercaptan, 13.8 g (0.1 mol) of potassium carbonate and 150 ml of dimethylformamide was stirred and heated at 90°C for 24 hours. The reaction mixture was then poured into 700 ml of ice water with stirring, and the resulting mixture was extracted three times with ether. The combined ether extract was washed with water and then with saturated sodium chloride, dried and concentrated leaving 26.02 g of an orange oil which tlc (1:4 ether:hexane) showed to contain 3 components. Sixteen grams of the material was purified on a silica gel dry column with 10 percent ether in hexane. There resulted from the center band 7.75 g of a yellow oil (52 percent of theory) whose nmr gave near theoretical integration. Distillation gave 5.23 g (35 percent of theory) of product, b.p. 172°-175°C/0.85 mm, as a yellow oil.

Analysis - calculated for $C_{15}H_{17}NS$ (%): C, 74.02; H, 7.04; N, 5.76; S, 13.18. Found (%): C, 74.0; H, 7.3; N, 5.7; S, 13.3.

Example 2

2-(2',5'-Dimethylbenzylsulfonyl)-4-methylpyridine N-oxide

A mixture of 10.0 g of 2-(2',5'-dimethylbenzylthio)-4-methylpyridine (crude material, 54 percent purity; 5.4 g real, 0.022 mol), 40 ml (0.40 mol) of 30 percent $H_2O_2$ and 200 ml of acetic acid was heated at 85°C for 15 1/2 hours. The resulting mixture was poured into 600 ml of ice water and extracted four times with chloroform. The chloroform extract was washed three times with saturated sodium bicarbonate, dried and concentrated leaving 6.62 g of a thick orange oil, which nmr showed to be approximately 65 percent product. This oil was stirred with 20 ml of ether, and the resulting precipitate was filtered off and washed with ether giving 2.56 g (40 percent of theory) of product as a tan solid; recrystallization from ethanol/$H_2O$ (1:2) yielded a white solid, m.p. 133.5°-135.5°C.

Analysis - calculated for $C_{15}H_{17}NO_3S$ (%): C, 61.83; H, 5.88; N, 4.81; S, 11.01. Found (%): C, 61.7; H, 5.9; N, 4.8; S, 10.9.

Example 3

2-(2',5'-Dimethylbenzylsulfonyl)-3-methylpyridine N-oxide

A mixture of 11.0 g (.042 mol) of 2-(2,5-dimethylbenzylthio)-3-methylpyridine N-oxide, 150 ml of acetic acid and 13 ml (.13 mol) of 30 percent $H_2O_2$ was heated on a steam bath overnight. After 15 1/2 hours, the reaction mixture was poured into 800 ml of ice water with stirring. The resulting precipitate was filtered off and washed with water and air dried. The resulting white solid was recrystallized from 200 ml of ethanol giving 8.68 g (71 percent of theory) of the product as a white solid, m.p. 174.5°-176°C.

Analysis - calculated for $C_{15}H_{17}NO_3S$ (%): C, 61.83; H, 5.88; N, 4.81; S, 11.01. Found (%): C, 61.8; H, 6.1; N, 4.6; S, 11.0.

Example 4

2-Chloro-4-phenylpyridine

A mixture of 70 g (0.4 mol) of 4-phenylpyridine N-oxide (Aldrich Chemical Co.), 250 g (4.2 mol) of sodium chloride and 450 ml (4.92 mol) of phosphorous oxychloride was stirred and warmed on a steam bath. After 17 hours, a distillation head was put on the reaction flask, and 375 ml of $POCl_3$ was

distilled from the reaction mixture. The cooled residue was then cautiously diluted with ca. 200 ml of water and cooled in an ice bath. It was then made basic by the slow addition of 275 ml of 50 percent KOH. The resulting solid was filtered from the ice-cooled reaction mixture and washed with water giving a wet, brick red solid which was taken up in chloroform, dried and concentrated. There resulted 68.27 g of a brick red solid (90 percent of theory). This was purified on a 3-in. silica gel dry column with chloroform. The front-running band was collected giving 54.07 g (75 percent of theory) of the product as a tan solid, m.p. 66°-68°C.

Analysis - calculated for $C_{11}H_8ClN$ (%): C, 69.66; H, 4.25; N, 7.39. Found (%): C, 69.5; H, 4.1; N, 7.1.

Example 5

2-(2',5'-Dimethylbenzylthio)-4-phenylpyridine

To a slurry of 16.83 g (.15 mol) of potassium t-butoxide in 200 ml of DMSO in a $N_2$ atmosphere was added 20.9 ml (.18 mol) of 2,5-dimethylbenzylmercaptan (ca. 75 percent pure), followed by 28.60 g (.15 mol) of 2-chloro-4-phenylpyridine. The reaction mixture was stirred at room temperature for 3 days. It was then diluted with 400 ml of water and extracted with four 100 ml portions of ether. The combined ether extract was washed four times with water and then with saturated sodium chloride. It was dried and concentrated giving 41.3 g of an orange oil, which was purified on a 3-in. silica gel dry column with 6:1 hexane:ether.

There resulted 16.56 g of a light yellow solid. 5.4 g was recrystallized from 15 ml of ethanol giving 4.91 g (34 percent of theory) of a white crystalline solid, m.p. 66°-68°C.

Analysis - calculated for $C_{20}H_{19}NS$ (%): C, 78.64; H, 6.27; N, 4.59; S, 10.50. Found (%): C, 78.7, 78.6; H, 6.6, 6.5; N, 4.1, 4.1; S, 9.4.

Example 6

2-(2',5'-Dimethylbenzylsulfonyl)-4-phenylpyridine N-oxide

A mixture of 10 g (.033 mol) of 2-(2',5'-dimethylbenzylthio)-4-phenylpyridine, 36 ml (.36 mol) of 30 percent hydrogen peroxide and 250 ml of acetic acid was warmed on the steam bath for 21 hours. The hot reaction mixture was poured into 2000 ml of ice water with stirring. After 1 hour, the

reaction mixture was filtered, and the precipitate was washed with water and air dried giving 5.98 g of a yellow solid. Purification on a 2-in. silica gel dry column with 8:1 methylene chloride:acetone, followed by recrystallization from 50 ml of ethanol, gave 3.10 g (24 percent of theory) of a light yellow solid, m.p., 156°-157°C.

Analysis - calculated for $C_{20}H_{19}NO_3S$ (%): C, 67.94; H, 5.42; N, 3.96; S, 9.07. Found (%): C, 67.7; H, 5.4; N, 4.0; S, 9.0.

## Example 7

## 2-Chloro-4-t-butylpyridine

A mixture of 22.69 g (.15 mol) of 4-t-butylpyridine N-oxide, 91.5 ml (1.0 mol) of phosphorous oxychloride and 50 ml of chloroform was heated at reflux for 6 hours and then held overnight at room temperature. The reaction mixture was concentrated to 50 ml, and the residue was slowly poured into 1100 ml of water with stirring. The resultant mixture was neutralized with sodium carbonate and then extracted with four 100 ml portions of chloroform. The combined extract was washed with saturated NaCl, dried and concentrated leaving 25.11 g of an orange oil. This oil was purified on a silica gel dry column with 1:8 acetone:hexane. The fast moving major band gave 14.45 g (56 percent of theory) of a yellow oil.

## Example 8

## 2-(2',5'-Dimethylbenzylthio)-4-t-butylpyridine

To a solution of sodium methoxide, prepared from 1.68 g (.073 g atom) of sodium and 35 ml of methanol, was added 11.10 g (.073 mol) of 2,5-dimethylbenzylmercaptan, and the resulting mixture was heated at reflux for 1 hour, followed by concentration to dryness. To the residue was added 45 ml of tetrahydrofuran followed by 12.46 g (.073 mol) of the compound of Example 7. The resulting mixture was heated at reflux for 4 days. It was then poured into 600 ml of water and extracted with five portions of ether. The combined extract was washed with sat. NaCl, dried and concentrated leaving 21.64 g (104 percent of theory) of an orange oil. Purification on a silica gel dry column ($CH_2Cl_2$) gave 10.43 g (50 percent of theory) of a yellow oil. Nmr was consistent with the desired product.

## Example 9

## 2-(2',5'-Dimethylbenzylsulfonyl)-4-t-butylpyridine N-oxide

A mixture of 10.18 g (.036 mol) of the compound of Example 8, 36 ml (.36 mol) of 30 percent hydrogen peroxide and 250 ml of acetic acid was warmed on a steam bath overnight. The resulting mixture was poured into 2000 ml of water and then cooled by the addition of ice. The light yellow solid was removed by filtration and water washed giving 5.22 g (43 percent of theory). Purification on a silica dry column (ether) gave 4.28 g of a white solid, m.p. 174.5°-176°C. 3.7 g was recrystallized from 50 ml of ethanol giving 3.48 g of a white crystalline solid (33 percent of theory), m.p. 175.5°-176°C.

Analysis - calculated for $C_{18}H_{23}NO_3S$ (%): C, 64.83; H, 6.95; N, 4.20; S, 9.62. Found (%): C, 65.0; H, 7.3; N, 4.2; S, 9.8.

## Example 10

## 2-(2',5'-Dimethylbenzylthio)-4-cyanopyridine N-oxide

Into a flame dried flask, in a $N_2$ atmosphere, was introduced a solution of 24.02 g (.2 mol) of 4-cyanopyridine N-oxide (Pfaltz and Bauer) in 600 ml of dry tetrahydrofuran. The solution was cooled to –76°C with a dry ice-acetone bath. To this stirred solution was added dropwise over 22 min. a solution of 1.8 M-n-butyllithium in hexane (111.2 ml, .2 mol). The red reaction mixture was stirred at –76°C for 30 min. 6.4 g (.2 mol) of sulfur was then added, and stirring was continued at –76°C for another 30 min. A solution of 26.7 ml (.2 mol) of 2,5-dimethylbenzylchloride (Aldrich) in 20 ml of tetrahydrofuran was then added dropwise, and stirring at –76°C was continued for 1 hour, after which the reaction mixture was allowed to warm to room temperature overnight. It was then poured into 3 liter of water and extracted with methylene chloride and ether. The combined extract was washed with water, dried and concentrated giving 46.6 g of a thick, red tar whose nmr was consistent with the desired product plus solvents. 18.0 g of this material was purified by dry column chromatography (silica gel/ether) giving 5.21 g (30 percent of theory) of a brown solid which was shown by tlc to contain two components.

Example 11

2-(2',5'-Dimethylbenzylsulfonyl)-4-cyanopyridine N-oxide

A mixture of 12.65 g (.053 mol) of the compound of Example 10 (impure), 50 ml (0.5 mol) of 30 percent hydrogen peroxide and 350 ml of acetic acid was warmed on a steam bath overnight. The reaction mixture was poured into 2500 ml of water and extracted with methylene chloride. The combined extract was washed with sodium carbonate until no more gas was evolved. It was then dried and concentrated leaving 3.84 g of a gummy yellow solid. Purification on a silica gel dry column ($CH_2Cl_2$) followed by recrystallization from 10 ml of acetonitrile plus 25 ml of ethanol gave 1.0 g of a white crystalline solid (7 percent of theory), m.p. 198°-199°C.

Analysis - calculated for $C_{15}H_{14}N_2O_3S$ (%): C, 59.58; H, 4.67; N, 9.27; S, 10.61. Found (%): C, 58.6; H, 4.7: N, 9.2; S, 10.5.

Example 12

6-Chloro-2-picoline N-oxide

Following the procedure of T. Kato and T. Niitsuma, Heterocycles, 1, 233 (1973), the title compound is prepared as follows.

A mixture of 61.46 g (.48 mol) of 6-chloro-2-picoline (Aldrich), 100 g (0.49 mol) of m-chloroperbenzoic acid (Aldrich, 85 percent) and 750 ml of chloroform was stirred at room temperature for 4 days. The reaction mixture was then cooled in an ice bath and filtered, and the solid m-chlorobenzoic acid was washed with chloroform. The yellow filtrate was washed with four 100 ml portions of 5 N NaOH followed by sat. NaCl. It was then dried and concentrated leaving 57.72 g of an orange oil which could not be distilled (darkened). The oil was purified on a dry column, developing with ether and gradually changing to 25 percent acetone in ether. This gave a main fraction containing 27.98 g of an orange oil. A second fraction gave 9.86 g of an orange oil which had identical spectral characteristics.

Example 13

2-(2',5'-Dimethylbenzylthio)-6-methylpyridine N-oxide

A mixture of 10 g (.07 mol) of 6-chloro-2-picoline N-oxide, 11 ml (.067 mol) of 2,5-dimethylbenzyl mercaptan (95 percent pure), 10 ml (.07 mol) of

triethylamine and 100 ml of dimethylformamide was stirred overnight at 90°C. It was then poured into 600 ml of ice water, and the resulting mixture stirred for 15 min. and filtered. There resulted 8.32 g (48 percent of theory) of a white solid. An additional 0.95 g precipitated on standing. The combined solid was recrystallized from 140 ml of ethyl acetate and 280 ml of hexane, and the resulting tan needles were put on a silica gel dry column and developed with ether. The band of $R_f$ ca. 0.1-0.3 gave 4.07 g of a white solid. This was recrystallized from 29 ml of ethyl acetate, giving 3.16 g of white needles, m.p. 88-112°C. This was taken up in 35 ml of $CHCl_3$, dried over $MgSO_4$ and precipitated with 200 ml of pentane giving 1.52 g of a white solid, m.p. 87.5°-110°C.

## Example 14

## 2-(2',5'-dimethylbenzylsulfonyl)-6-methylpyridine N-oxide

A mixture of 13.05 g (.05 mol) of 2-(2',5'-dimethylbenzylthio)-6-methylpyridine N-oxide, 25 ml (.25 mol) of 30 percent $H_2O_2$ and 200 ml of acetic acid was stirred and warmed on a steam bath for 3 hours. This mixture was then poured into 1000 ml of a stirred ice-water mixture. Filtration and air drying gave 6.45 g of a yellow powder. Recrystallization from 400 ml of ethanol gave 4.73 g (32 percent of theory) of a white solid, m.p. 190°-192°C.

Analysis - calculated for $C_{15}H_{17}NO_3S$ (%): C, 61.83; H, 5.88; N, 4.81; S, 11.01. Found (%): C, 61.7; H, 6.1; N, 4.8; S, 11.9.

## Example 15

## 2-(2',5'-Dimethylbenzylthio)-4-methylpyridine N-oxide

To a stirred mixture of 10.52 g (.073 mol) of 2-chloro-4-methylpyridine N-oxide (prepared from 2-chloropicoline and mcpba in chloroform, 17 hours at 20°C), 12.11 ml (0.073 mol real) of 95 percent pure 2,5-dimethylbenzylmercaptan and 100 ml of dimethylformamide was added 10.22 ml (.073 mol) of triethylamine. The mixture was stirred at room temperature for 4 hours and then at 90°C for 3 hours. It was then cooled and poured into 500 ml of ice water with stirring. The resulting tan solid was filtered and water washed giving 9.58 g (51 percent of theory). Nmr indicated a second component without the pyridyl ring. This mixture was purified on a short silica column, eluting first

with 750 ml of ether, followed by 1 liter of acetone. The combined acetone fractions gave 5.22 g of a white solid (28 percent of theory). The center cut of the acetone fraction gave m.p. 176°-178°C.

Example 16

2-(2',5'-Dimethylbenzylsulfinyl)-4-methylpyridine N-oxide

A mixture of 6.7 g (.026 mol) of 2-(2',5'-dimethylbenzylthio)-4-methylpyridine N-oxide 5.28 g (.026 mol) of m-chloroperbenzoic acid (Aldrich) and 50 ml of methylene chloride (exotherm on mixing) was stirred for 4 days at room temperature. The reaction mixture was then cooled in an ice bath, filtered and the precipitate was washed with cold methylene chloride. The filtrate was washed twice with 5 percent NaOH and then with sat. NaCl, dried and concentrated leaving 6.75 g of a white foam which crystallized on standing. The residue was purified on a silica gel dry column with 1:4 acetone:ether. There resulted 1.42 g of a white solid, m.p. 125°-126.5°C.

Analysis - calculated for $C_{15}H_{17}NO_2S$ (%): C, 65.42; H, 6.22; N, 5.09. Found (%): C, 65.3; H, 6.5; N, 5.1.

Example 17A

2-(α-methyl-2',6'-Dichlorobenzylthio)-3-methylpyridine N-oxide

A solution of 6.2 g (.03 mol) of 2,6-dichloro-α-methylbenzyl mercaptan in 3 ml of methanol was slowly added to sodium methoxide prepared from 0.69 g (.03 g-atom) of sodium and 15 ml of methanol. The reaction mixture was heated at reflux for 1 hour. It was then concentrated at reduced pressure. The resulting white solid was slurried with 10 ml of tetrahydrofuran in a $N_2$ atmosphere. To this slurry was added all at once a slurry of 5.0 g (.027 mol) of 2-bromo-3-methylpyridine N-oxide in 15 ml of tetrahydrofuran. The reaction mixture warmed spontaneously to 42°C. The reaction mixture was stirred overnight and then poured into 150 ml of water. The resulting mixture of water plus yellow precipitate was extracted four times with methylene chloride. The combined extract was washed with saturated NaCl, dried and concentrated in vacuo leaving 8.73 g (103 percent of theory) of a yellow solid. Tlc (ether) indicated one major component with two very minor impurities. Infrared and $H^1$ NMR confirmed the product structure.

3.7 g was recrystallized from ethanol giving 2.47 g of yellow crystalline solid, m.p. 161.5°-163.5°C.

Analysis - calculated for $C_{14}H_{13}Cl_2NOS$ (%): C, 53.50; H, 4.17; N, 4.46. Found (%): C, 53.6, 53.4; H, 4.3, 4.3; N, 4.4, 4.4.

Example 17B

2-(α-methyl-2',6'-Dichlorobenzylsulfonyl)-3-methylpyridine N-oxide

A mixture of 5.0 g (.016 mol) of the compound of Example 17A, 16 ml (.16 mol) of 30 percent $H_2O_2$, and 100 ml of acetic acid was stirred and warmed on a steam bath for 17 hours. It was then poured into 700 ml of ice water, and the resulting mixture was extracted with three 100 ml portions of methylene chloride. The combined extract was washed three times with saturated $NaHCO_3$, dried, and concentrated at reduced pressure leaving 3.29 g of a yellow semi-solid. Purification on a silica gel dry column (1:9 acetone:$CH_2Cl_2$), followed by recrystallization from 30 ml of i-propanol plus 20 ml of methanol gave 0.73 g (13 percent of theory) of a white solid, m.p. 199.5°-200°C.

Analysis - calculated for $C_{14}H_{13}Cl_2NO_3S$ (%): C, 48.56; H, 3.78; N, 4.05. Found (%): C, 48.6, 48.6; H, 3.9, 3.9; N, 3.7, 4.0.

The following compounds were prepared utilizing synthesis methods analogous to the general methods set forth above as illustrated by the preceding specific examples.

Example 18 - 2-(2',5'-dimethylbenzylsulfonyl)pyridine N-oxide, m.p. 156°-158°C. (see Example 1, U.S. Patent No. 3,960,542)

Example 19 - 2-(2',5'-dimethylbenzylsulfonyl)-5-methylpyridine N-oxide, m.p. 155°-156°C.

Example 20 - 2-(2',5'-dimethylbenzylsulfonyl)-5-bromopyridine N-oxide, m.p. 191°-192°C.

Example 21 - 2-(2',5'-dimethylbenzylsulfonyl)-4-ethylpyridine N-oxide, m.p. 117°-118°C.

Example 22 - No example.

Example 23 - 2-(2',5'-dimethylbenzylsulfonyl)-4,5-dimethylpyridine N-oxide, m.p. 192.5°-193.5°C.

Example 24 - 2-(2',5'-dimethylbenzylsulfonyl)-3,4-dimethylpyridine N-oxide, m.p. 133°-134°C.

Example 25 - 2-(2',5'-dimethylbenzylsulfonyl)-4-chloropyridine N-oxide, m.p. 136°-138°C.

Example 26 - 2-(2'-methylbenzylsulfonyl)-4-methylpyridine N-oxide, m.p. 161°-162°C.

Example 27 - 2-(4'-chlorobenzylsulfonyl)-4-methylpyridine N-oxide, m.p. 180°-181°C.

Example 28 - 2-(4'-t-butylbenzylsulfonyl)-4-methylpyridine N-oxide, m.p. 162°-163°C.

Example 29 - 2-(4'-phenylbenzylsulfonyl)-4-methylpyridine N-oxide, m.p. 194°-195°C.

Example 30 - 2-(3'-trifluoromethylbenzylsulfonyl)-4-methylpyridine N-oxide, m.p. 145°-146°C.

Example 31 - 2-(4'-fluorobenzylsulfonyl)-4-methylpyridine N-oxide, m.p. 138°-139°C.

Example 32 - 2-(2',6'-dichlorobenzylsulfonyl)-4-methylpyridine N-oxide, m.p. 201°-202°C.

Example 33 - 2-(3'-cyanobenzylsulfonyl)-4-methylpyridine N-oxide, m.p. 150°-151°C.

Example 34 - 2-benzylsulfonyl-4-methylpyridine N-oxide, m.p. 144°-145°C.

Example 35 - 2-(4'-nitrobenzylsulfonyl)-4-methylpyridine N-oxide, m.p. 242°-243°C (dec.).

Example 36 - 2-(2',4',6'-trimethylbenzylsulfonyl)-4-methylpyridine N-oxide, m.p. 219°-220°C.

Example 37 - 2-(1'-naphthylmethylsulfonyl)-4-methylpyridine N-oxide, m.p. 116°-117°C (dec).

Example 38 - 2-(2',6'-dichlorobenzylsulfonyl)-5-bromopyridine N-oxide, m.p. 203°-204°C.

Example 39 - 2-($\alpha$,2',5'-trimethylbenzylsulfonyl)-4-methylpyridine N-oxide, m.p. 202°-203°C.

Example 40 - 2-(2',5'-dimethylbenzylsulfinyl)-3-methylpyridine N-oxide, m.p. 136°-140°C.

## Preemergence Herbicide Activity

To illustrate the preemergence herbicidal efficacy of the pyridine N-oxide compounds of the present invention, test formulations of representative

compounds are prepared by mixing 20 ml of an acetone solution containing 0.0416 g of the test compound with 20 ml of water containing 0.02 ml of Ortho X-77 surfactant. The resultant formulations contain 1040 ppm of test compound, 50 percent, by volume, of acetone and 0.05 percent, by volume, of surfactant. Appropriate lower concentrations are obtained by diluting this formulation with surfactant-acetone solution so that the concentration of adjuvants is maintained at the original levels.

Seeds of three broadleaf, three grassy weed species and three crop species are planted in the soil contained in 10 x 8 x 3 inch fiber pans filled with 2.0 inches of pasteurized soil (Kingsville sandy loam). The broadleaf species are pigweed (Amaranthus retroflexus L.), velvet leaf (Abutilon theophrasti Medic.) and mustard (Brassica kaber (DC.) Wheeler); the grasses are red millet (Panicum milliaceum L.), green foxtail (Setaria viridis (L.) Beauv.) and barnyard grass (Echinochloa crus-galli (L.) Beauv.), and the crops are cotton (Gossypium hirsutum L. 'Stoneville' 213), soybean (Glycine max (L.) Merr. 'Lancer') and corn (Zea mays (L.) 'Pioneer' 3518 F14). The pans are then sprayed so that the soil surface is uniformly covered with dilutions of the stock formulation providing dosage rates of the test compounds corresponding to 8, 4, 2, 1, 0.5, 0.25, 0.12, 0.06, etc., kg/hectare. Two weeks after treatment, percent control (plant kill) at each dosage rate is estimated.

In Table I below, the results obtained according to the foregoing test procedures with respect to the application or dosage rate of the active compounds according to the present invention and the percent weed control (effectiveness) exerted by the test compounds are summarized. Also, shown is percent control (tolerance) on crops.

Table I

% Control Preemergence[1]

| Compound Example No. | DOSE KG/HA | PIG-WEED | VEL. LEAF | MUS-TARD | RED MIL. | FOX-TAIL | B.Y. GRSS | COT-TON | SOY-BEAN | CORN |
|---|---|---|---|---|---|---|---|---|---|---|
| 2 | 4 | 95 | 35 | 78 | 91 | 91 | 95 | 25 | 75 | 88 |
| | 2 | 86 | 14 | 71 | 86 | 88 | 94 | 7 | 57 | 77 |
| | 1 | 79 | 12 | 48 | 72 | 78 | 90 | 0 | 36 | 58 |
| | .5 | 70 | 20 | 10 | 30 | 60 | 95 | 0 | 50 | 30 |
| 3 | 8 | 50 | 20 | 60 | 90 | 90 | 95 | 0 | 10 | 90 |
| | 4 | 80 | 10 | 60 | 90 | 80 | 90 | 0 | 0 | 70 |
| | 2 | 70 | 0 | 20 | 80 | 80 | 90 | 0 | 0 | 80 |
| | 1 | 70 | 0 | 10 | 60 | 70 | 90 | 0 | 20 | 20 |
| | .5 | 30 | 10 | 20 | 30 | 40 | 70 | 0 | 0 | 0 |
| | .25 | 0 | 0 | 10 | 10 | 0 | 10 | 0 | 0 | 0 |
| 6 | 8 | 100 | 60 | 10 | 70 | 90 | 100 | 10 | 30 | 30 |
| | 4 | 60 | 7 | 20 | 50 | 87 | 90 | 10 | 13 | 13 |
| | 2 | 43 | 7 | 7 | 37 | 77 | 87 | 10 | 20 | 0 |
| | 1 | 27 | 3 | 0 | 23 | 63 | 67 | 3 | 3 | 0 |
| | .5 | 33 | 0 | 0 | 7 | 20 | 17 | 0 | 0 | 3 |
| | .25 | 7 | 0 | 0 | 3 | 7 | 3 | 3 | 0 | 3 |
| | .12 | 10 | 0 | 0 | 0 | 0 | 10 | 0 | 0 | 0 |
| 9 | 8 | 100 | 20 | 90 | 100 | 100 | 100 | 0 | 90 | 90 |
| | 4 | 60 | 40 | 90 | 95 | 100 | 100 | 30 | 90 | 90 |
| | 2 | 80 | 20 | 80 | 90 | 90 | 95 | 20 | 80 | 50 |
| | 1 | 40 | 10 | 80 | 90 | 90 | 95 | 0 | 80 | 70 |
| | .5 | 20 | 0 | 70 | 90 | 90 | 90 | 10 | 70 | 30 |
| | .25 | 0 | 0 | 20 | 80 | 90 | 100 | 0 | 40 | 20 |
| | .12 | 0 | 0 | 50 | 0 | 20 | 50 | 20 | 0 | 0 |
| | .06 | 0 | 0 | 0 | 0 | 0 | 30 | 0 | 0 | 0 |
| | .03 | 0 | 0 | 20 | 0 | 0 | 20 | 0 | 0 | 0 |
| 11 | 8 | 20 | 0 | 0 | 10 | 60 | 80 | 0 | 0 | 0 |
| | 4 | 30 | 0 | 0 | 10 | 10 | 40 | 0 | 10 | 0 |
| 16 | 8 | 100 | 60 | 70 | 95 | 95 | 95 | 10 | 70 | 90 |
| | 4 | 80 | 20 | 60 | 95 | 90 | 95 | 0 | 70 | 70 |
| | 2 | 40 | 0 | 60 | 90 | 90 | 90 | 10 | 60 | 70 |
| | 1 | 15 | 5 | 20 | 80 | 90 | 80 | 0 | 25 | 55 |
| | .5 | 0 | 0 | 0 | 40 | 50 | 80 | 0 | 10 | 30 |
| | .25 | 0 | 0 | 0 | 10 | 70 | 70 | 0 | 0 | 20 |
| | .12 | 0 | 0 | 0 | 0 | 10 | 10 | 0 | 10 | 0 |

Table I (continued)

| | | | | | | | | | | |
|---|---|---|---|---|---|---|---|---|---|---|
| 18[2] | 4 | 95 | 90 | 80 | 90 | 90 | 95 | 10 | 60 | 95 |
| | 2 | 90 | 80 | 60 | 90 | 90 | 95 | 10 | 50 | 80 |
| | 1 | 60 | 35 | 60 | 85 | 85 | 93 | 5 | 25 | 75 |
| | .5 | 60 | 30 | 10 | 30 | 70 | 80 | 0 | 30 | 50 |
| 19 | 8 | 60 | 50 | 40 | 60 | 90 | 90 | 0 | 60 | 50 |
| | 4 | 10 | 0 | 10 | 30 | 60 | 90 | 0 | 30 | 0 |
| | 2 | 10 | 10 | 10 | 20 | 70 | 90 | 0 | 20 | 0 |
| | 1 | 0 | 0 | 10 | 0 | 10 | 10 | 10 | 10 | 0 |
| 20 | 8 | 50 | 10 | 10 | 80 | 80 | 90 | 10 | 50 | 50 |
| | 4 | 60 | 10 | 10 | 70 | 60 | 80 | 0 | 0 | 60 |
| | 2 | 50 | 0 | 10 | 70 | 30 | 90 | 0 | 0 | 70 |
| | 1 | 20 | 0 | 10 | 90 | 60 | 90 | 0 | 0 | 60 |
| | .5 | 10 | 0 | 0 | 90 | 50 | 90 | 0 | 0 | 40 |
| | .25 | 0 | 0 | 30 | 80 | 70 | 90 | 10 | 0 | 30 |
| | .12 | 0 | 10 | 20 | 40 | 60 | 80 | 10 | 0 | 0 |
| | .06 | 0 | 0 | 0 | 0 | 10 | 20 | 0 | 20 | 10 |
| | .03 | 0 | 0 | 0 | 0 | 0 | 10 | 0 | 0 | 0 |
| 21 | 8 | 95 | 20 | 90 | 100 | 95 | 100 | 20 | 70 | 90 |
| | 4 | 100 | 20 | 70 | 100 | 95 | 100 | 0 | 70 | 95 |
| | 2 | 100 | 10 | 70 | 95 | 95 | 100 | 20 | 30 | 90 |
| | 1 | 80 | 0 | 60 | 90 | 90 | 95 | 0 | 0 | 70 |
| | .5 | 40 | 0 | 40 | 90 | 90 | 95 | 0 | 0 | 10 |
| | .25 | 20 | 0 | 30 | 20 | 60 | 50 | 0 | 10 | 0 |
| | .12 | 10 | 0 | 0 | 10 | 40 | 40 | 0 | 20 | 0 |
| | .06 | 0 | 0 | 0 | 0 | 20 | 40 | 0 | 20 | 0 |
| 23 | 8 | 100 | 0 | 40 | 30 | 80 | 70 | 0 | 30 | 10 |
| | 4 | 67 | 13 | 3 | 30 | 80 | 53 | 3 | 20 | 3 |
| | 2 | 43 | 10 | 3 | 17 | 43 | 33 | 0 | 13 | 3 |
| | 1 | 37 | 7 | 3 | 7 | 20 | 27 | 3 | 7 | 0 |
| | .5 | 10 | 3 | 3 | 0 | 0 | 0 | 0 | 3 | 0 |
| | .25 | 30 | 3 | 20 | 7 | 7 | 7 | 7 | 13 | 3 |
| | .12 | 90 | 0 | 20 | 10 | 20 | 0 | 0 | 10 | 0 |

Table I (continued)

| | | | | | | | | | | |
|---|---|---|---|---|---|---|---|---|---|---|
| 24 | 8 | 80 | 0 | 90 | 100 | 100 | 100 | 30 | 40 | 95 |
| | 4 | 70 | 10 | 70 | 95 | 98 | 100 | 5 | 40 | 75 |
| | 2 | 40 | 15 | 30 | 85 | 88 | 98 | 5 | 20 | 60 |
| | 1 | 45 | 10 | 30 | 65 | 80 | 90 | 15 | 25 | 35 |
| | .5 | 65 | 0 | 20 | 30 | 55 | 65 | 15 | 10 | 5 |
| | .25 | 20 | 5 | 0 | 0 | 10 | 40 | 0 | 15 | 0 |
| | .12 | 0 | 0 | 0 | 0 | 0 | 20 | 0 | 0 | 0 |
| | .06 | 0 | 0 | 0 | 0 | 0 | 0 | 0 | 0 | 0 |
| 25 | 8 | 100 | 80 | 70 | 95 | 90 | 95 | 0 | 0 | 0 |
| | 4 | 95 | 10 | 10 | 80 | 60 | 90 | 0 | 0 | 0 |
| | 2 | 90 | 0 | 20 | 90 | 70 | 90 | 0 | 20 | 0 |
| | 1 | 80 | 15 | 30 | 80 | 65 | 90 | 25 | 20 | 5 |
| | .5 | 65 | 10 | 20 | 85 | 75 | 90 | 30 | 0 | 10 |
| | .25 | 70 | 0 | 0 | 70 | 90 | 80 | 40 | 0 | 0 |
| | .12 | 20 | 0 | 10 | 60 | 80 | 80 | 10 | 0 | 10 |
| | .06 | 0 | 0 | 0 | 50 | 70 | 70 | 40 | 0 | 0 |
| | .03 | 40 | 0 | 0 | 40 | 60 | 0 | 20 | 0 | 0 |
| 26 | 8 | 95 | 40 | 90 | 90 | 90 | 95 | 30 | 60 | 90 |
| | 4 | 100 | 30 | 95 | 95 | 95 | 95 | 10 | 40 | 90 |
| | 2 | 100 | 10 | 90 | 90 | 90 | 95 | 20 | 40 | 70 |
| | 1 | 10 | 0 | 70 | 80 | 90 | 95 | 10 | 20 | 50 |
| | .5 | 20 | 0 | 70 | 50 | 70 | 70 | 10 | 20 | 10 |
| | .25 | 0 | 0 | 10 | 30 | 80 | 70 | 0 | 10 | 0 |
| | .12 | 0 | 0 | 10 | 0 | 0 | 20 | 0 | 20 | 0 |
| 27 | 8 | 100 | 10 | 80 | 100 | 90 | 100 | 0 | 30 | 90 |
| | 4 | 40 | 0 | 30 | 90 | 90 | 100 | 0 | 0 | 70 |
| | 2 | 70 | 0 | 30 | 95 | 90 | 90 | 0 | 20 | 80 |
| | 1 | 30 | 10 | 0 | 40 | 50 | 90 | 0 | 0 | 20 |
| | .5 | 0 | 0 | 0 | 40 | 60 | 80 | 0 | 0 | 0 |
| | .25 | 0 | 0 | 10 | 10 | 0 | 0 | 10 | 10 | 0 |
| | .12 | 0 | 0 | 0 | 0 | 0 | 0 | 0 | 10 | 0 |
| 28 | 8 | 20 | 0 | 40 | 70 | 70 | 90 | 0 | 0 | 40 |
| | 4 | 0 | 0 | 0 | 60 | 90 | 90 | 30 | 0 | 0 |
| | 2 | 20 | 0 | 0 | 10 | 60 | 80 | 10 | 0 | 0 |
| | 1 | 10 | 0 | 0 | 0 | 0 | 0 | 0 | 0 | 0 |
| | .5 | 0 | 0 | 0 | 0 | 0 | 0 | 0 | 10 | 0 |

Table I (continued)

| | | | | | | | | | | |
|---|---|---|---|---|---|---|---|---|---|---|
| 29 | 8 | 30 | 10 | 40 | 30 | 80 | 60 | 0 | 20 | 0 |
| | 4 | 20 | 10 | 30 | 30 | 70 | 70 | 0 | 0 | 0 |
| | 2 | 50 | 10 | 40 | 30 | 80 | 20 | 40 | 0 | 0 |
| | 1 | 10 | 0 | 20 | 10 | 40 | 70 | 10 | 0 | 0 |
| | .5 | 0 | 0 | 0 | 20 | 40 | 10 | 10 | 10 | 0 |
| 30 | 8 | 80 | 30 | 60 | 90 | 90 | 90 | 20 | 20 | 40 |
| | 4 | 100 | 0 | 20 | 95 | 90 | 90 | 20 | 40 | 80 |
| | 2 | 70 | 0 | 20 | 80 | 80 | 90 | 0 | 20 | 10 |
| | 1 | 0 | 10 | 20 | 30 | 60 | 60 | 10 | 10 | 0 |
| | .5 | 0 | 0 | 20 | 20 | 10 | 10 | 10 | 20 | 0 |
| 31 | 8 | 70 | 20 | 40 | 90 | 90 | 100 | 40 | 50 | 60 |
| | 4 | 70 | 10 | 30 | 90 | 90 | 90 | 20 | 20 | 40 |
| | 2 | 40 | 10 | 20 | 60 | 90 | 90 | 20 | 10 | 10 |
| | 1 | 10 | 20 | 30 | 70 | 70 | 50 | 20 | 50 | 20 |
| | .5 | 10 | 10 | 20 | 30 | 30 | 20 | 10 | 20 | 10 |
| 32 | 8 | 100 | 40 | 90 | 95 | 95 | 95 | 30 | 90 | 90 |
| | 4 | 98 | 50 | 95 | 100 | 98 | 100 | 27 | 85 | 83 |
| | 2 | 97 | 37 | 90 | 95 | 95 | 100 | 23 | 63 | 80 |
| | 1 | 75 | 25 | 90 | 95 | 93 | 98 | 15 | 55 | 55 |
| | .5 | 50 | 0 | 75 | 90 | 85 | 95 | 0 | 20 | 30 |
| | .25 | 75 | 5 | 65 | 85 | 90 | 98 | 0 | 15 | 40 |
| | .12 | 58 | 7 | 13 | 57 | 73 | 72 | 3 | 7 | 3 |
| | .06 | 27 | 7 | 13 | 43 | 53 | 70 | 3 | 13 | 7 |
| | .03 | 25 | 0 | 5 | 5 | 30 | 45 | 0 | 0 | 0 |
| 33 | 8 | 100 | 80 | 50 | 80 | 95 | 100 | 10 | 20 | 80 |
| | 4 | 92 | 10 | 17 | 83 | 90 | 90 | 10 | 3 | 30 |
| | 2 | 75 | 17 | 3 | 63 | 83 | 80 | 13 | 3 | 7 |
| | 1 | 50 | 10 | 3 | 37 | 60 | 40 | 7 | 3 | 0 |
| | .5 | 25 | 0 | 5 | 25 | 15 | 5 | 5 | 0 | 0 |
| | .25 | 20 | 0 | 0 | 23 | 17 | 3 | 17 | 7 | 0 |
| | .12 | 40 | 0 | 60 | 10 | 0 | 10 | 0 | 0 | 0 |
| 34 | 8 | 80 | 80 | 70 | 90 | 95 | 100 | 20 | 70 | 80 |
| | 4 | 62 | 17 | 33 | 88 | 97 | 95 | 7 | 27 | 67 |
| | 2 | 60 | 10 | 23 | 77 | 87 | 90 | 7 | 20 | 30 |
| | 1 | 50 | 13 | 10 | 57 | 73 | 80 | 13 | 13 | 7 |
| | .5 | 52 | 0 | 3 | 37 | 43 | 40 | 3 | 0 | 0 |
| | .25 | 57 | 3 | 23 | 27 | 20 | 7 | 3 | 0 | 0 |
| | .12 | 0 | 0 | 0 | 0 | 0 | 0 | 0 | 0 | 0 |
| | .06 | 30 | 0 | 0 | 20 | 20 | 10 | 10 | 0 | 0 |

Table I (continued)

| | | | | | | | | | | |
|---|---|---|---|---|---|---|---|---|---|---|
| 35 | 8 | 100 | 10 | 40 | 80 | 90 | 95 | 0 | 0 | 0 |
| | 4 | 90 | 0 | 0 | 80 | 70 | 90 | 0 | 0 | 10 |
| | 2 | 70 | 0 | 10 | 30 | 50 | 90 | 0 | 10 | 0 |
| | 1 | 10 | 0 | 0 | 0 | 10 | 0 | 0 | 0 | 0 |
| 36 | 8 | 100 | 60 | 90 | 100 | 100 | 100 | 40 | 80 | 90 |
| | 4 | 100 | 60 | 90 | 100 | 100 | 100 | 50 | 70 | 90 |
| | 2 | 100 | 60 | 70 | 100 | 100 | 100 | 0 | 70 | 80 |
| | 1 | 80 | 35 | 45 | 95 | 95 | 100 | 25 | 60 | 70 |
| | .5 | 58 | 10 | 20 | 80 | 85 | 95 | 5 | 30 | 60 |
| | .25 | 50 | 10 | 20 | 70 | 80 | 90 | 10 | 20 | 40 |
| | .12 | 10 | 0 | 10 | 60 | 70 | 60 | 0 | 20 | 10 |
| | .06 | 20 | 0 | 0 | 40 | 50 | 30 | 0 | 20 | 0 |
| 38 | 8 | 60 | 20 | 50 | 90 | 70 | 90 | 30 | 30 | 40 |
| | 4 | 50 | 30 | 80 | 95 | 90 | 90 | 0 | 30 | 30 |
| | 2 | 70 | 50 | 60 | 95 | 90 | 90 | 0 | 20 | 20 |
| | 1 | 70 | 50 | 50 | 90 | 90 | 90 | 10 | 30 | 40 |
| | .5 | 20 | 0 | 10 | 60 | 50 | 90 | 0 | 0 | 20 |
| | .25 | 20 | 0 | 10 | 80 | 70 | 90 | 0 | 0 | 0 |
| | .12 | 0 | 0 | 10 | 20 | 10 | 70 | 0 | 0 | 0 |
| | .06 | 0 | 0 | 0 | 10 | 0 | 10 | 0 | 0 | 0 |
| 39 | 8 | 95 | 80 | 90 | 100 | 100 | 100 | 20 | 90 | 100 |
| | 4 | 100 | 90 | 80 | 100 | 100 | 100 | 60 | 70 | 90 |
| | 2 | 100 | 80 | 80 | 100 | 100 | 100 | 50 | 60 | 90 |
| | 1 | 70 | 50 | 70 | 100 | 100 | 100 | 40 | 60 | 90 |
| | .5 | 70 | 10 | 50 | 95 | 100 | 100 | 30 | 50 | 80 |
| | .25 | 20 | 0 | 20 | 90 | 100 | 100 | 10 | 20 | 50 |
| | .12 | 20 | 40 | 20 | 70 | 90 | 95 | 40 | 0 | 20 |
| 40 | 8 | 80 | 30 | 10 | 50 | 90 | 95 | 0 | 60 | 60 |
| | 4 | 60 | 0 | 40 | 90 | 90 | 90 | 0 | 60 | 10 |
| | 2 | 10 | 0 | 10 | 70 | 80 | 80 | 0 | 10 | 30 |
| | 1 | 0 | 0 | 10 | 20 | 80 | 80 | 0 | 20 | 20 |
| | .5 | 0 | 0 | 0 | 0 | 60 | 60 | 0 | 10 | 0 |
| | .25 | 10 | 0 | 10 | 20 | 20 | 10 | 10 | 0 | 0 |
| | .12 | 20 | 0 | 20 | 10 | 10 | 0 | 10 | 0 | 10 |

[1] Mean of all replications at indicated dose.

[2] Comparative test compound of U.S. Pat. No. 3,960,542.

## Plant Growth Regulant Activity

The compounds of the invention were also evaluated relative to plant growth regulant activity against three plant species in a petri dish test. The test is divided into a primary test where 500 $\mu g$ and 100 $\mu g$ chemical per petri dish (100 x 25 mm, disposable) are tested simultaneously and a secondary test where 50 $\mu g$, 10 $\mu g$ and 1 $\mu g$ chemical per petri dish are tested simultaneously. Compounds which pass the primary test are further evaluated in the secondary test. A special petri dish test may also be run to evaluate particular test compounds, in which case all five rates, i.e., 500, 100, 50, 10 and 1 $\mu g$ per petri dish, are tested simultaneously.

According to the test procedure, three plant species are planted in a petri dish which has been treated with the test substance. The three species are as follows: 1) a mixture of approximately 50 percent light-sensitive and 50 percent light-insensitive lettuce (Lactuca sativa L. 'Grand Rapids'); 2) soft red winter wheat [Triticum aestivum L. (Aestivicum Group) 'Abe']; and 3) pasture-type perennial ryegrass (Lolium perenne L. 'Linn'). Wheat and perennial ryegrass seeds are surface-sterilized with 1 percent sodium hypochloride for 10 minutes and 5 minutes, respectively.

Each test compound is formulated in acetone and aliquots of the test formulation are placed on three layers of sterilized filter papers (Whatman No. 1, 8.26 cm diameter) in each petri dish. As soon as the acetone evaporates, 7 ml of deionized water are added into each petri dish with an appropriate automatic dispenser. Then, 5 to 8 wheat seeds, 10 to 15 perennial ryegrass seeds and 10 to 15 lettuce seeds are placed on the filter paper of each petri dish. Dishes are then covered and seeds are germinated for 3 days at 20$^{o}$C at a relative humidity of 65 in the dark. The dishes are then removed from the dark growth chamber and maintained in lighted environmental growth chambers for 4 days.

At the end of the seventh day of planting, growth and developmental responses/characteristics are evaluated.

The results are summarized in Table II.

Table II

| Example No. | Rates (ug/dish) | Test Species[1] Lettuce Root | Lettuce Stem | Wheat Root | Wheat Leaf | Perennial Ryegrass Root | Perennial Ryegrass Leaf |
|---|---|---|---|---|---|---|---|
| 2 | 500 | -3 | -3 | -3 | -3 | -3 | -3 |
| | 100 | 0 | -1 | -3 | -3 | -3 | -3 |
| | 50 | +2 | -1 | -2 | -1 | -2 | -3 |
| | 10 | +2 | 0 | +1 | -1 | 0 | -3 |
| | 1 | 0 | 0 | 0 | 0 | 0 | 0 |
| 3 | 500 | -2 | -3 | -3 | -3 | -3 | -3 |
| | 100 | 0 | -3 | -2 | -3 | -3 | -3 |
| | 50 | 0 | 0 | -2 | -3 | -3 | -3 |
| | 10 | 0 | 0 | 0 | 0 | 0 | -3 |
| | 1 | 0 | 0 | 0 | 0 | 0 | 0 |
| 6 | 500 | -1 | -3 | -2 | -3 | -2 | -3 |
| | 100 | 0 | -2 | -1 | -3 | -1 | -3 |
| | 50 | 0 | -1 | 0 | -1 | 0 | -2 |
| | 10 | 0 | 0 | 0 | 0 | 0 | -1 |
| | 1 | 0 | 0 | 0 | 0 | 0 | 0 |
| 9 | 500 | -1 | -2 | -3 | -3 | -3 | -3 |
| | 100 | 0 | -1 | -3 | -3 | -3 | -3 |
| | 50 | 0 | -1 | -3 | -3 | -3 | -3 |
| | 10 | +1 | -1 | -2 | -2 | -2 | -3 |
| | 1 | 0 | 0 | 0 | 0 | -1 | -2 |
| 16 | 500 | -3 | -3 | -3 | -3 | -3 | -3 |
| | 100 | 0 | -3 | -3 | -3 | -3 | -3 |
| | 50 | 0 | 0 | -2 | -3 | -3 | -3 |
| | 10 | 0 | 0 | 0 | -3 | 0 | -3 |
| | 1 | 0 | 0 | 0 | 0 | +2 | -2 |

Table II (continued)

| | | | | | | | |
|---|---|---|---|---|---|---|---|
| 17 | 500 | -2 | -3 | -3 | -3 | -3 | -3 |
| | 100 | -2 | -3 | -2 | -3 | -3 | -3 |
| | 50 | -1 | -1 | -2 | -2 | -3 | -3 |
| | 10 | 0 | 0 | 0 | 0 | -1 | -3 |
| | 1 | 0 | 0 | 0 | 0 | 0 | 0 |
| 18 | 500 | -2 | -3 | -3 | -3 | -3 | -3 |
| | 100 | -1 | -2 | -3 | -3 | -3 | -3 |
| | 50 | -2 | -2 | -3 | -3 | -3 | -3 |
| | 10 | 0 | -2 | -2 | -3 | -3 | -3 |
| | 1 | 0 | 0 | 0 | 0 | -2 | -2 |
| 19 | 500 | -3 | -3 | -2 | -3 | -3 | -3 |
| | 100 | -1 | -1 | -2 | -3 | -2 | -3 |
| | 50 | 0 | 0 | -1 | -1 | 0 | -3 |
| | 10 | 0 | 0 | 0 | 0 | 0 | -3 |
| | 1 | 0 | 0 | 0 | 0 | 0 | 0 |
| 20 | 500 | -3 | -3 | -2 | -2 | -3 | -3 |
| | 100 | -2 | -2 | -1 | -1 | -3 | -3 |
| | 50 | -3 | -2 | -2 | -2 | -3 | -3 |
| | 10 | -2 | -2 | 0 | 0 | -1 | -3 |
| | 1 | 0 | 0 | 0 | 0 | 0 | -2 |
| 21 | 500 | -3 | -3 | -3 | -3 | -3 | -3 |
| | 100 | -1 | -3 | -2 | -3 | -3 | -3 |
| | 50 | 0 | -3 | -2 | -3 | -3 | -3 |
| | 10 | 0 | -1 | -2 | -2 | -3 | -3 |
| | 1 | 0 | 0 | 0 | 0 | 0 | 0 |

Table II (continued)

| | | | | | | | |
|---|---|---|---|---|---|---|---|
| 23 | 500 | −2 | −3 | −3 | −2 | −3 | −3 |
| | 100 | 0 | 0 | −3 | −2 | −3 | −3 |
| | 50 | 0 | 0 | −3 | −1 | −2 | −1 |
| | 10 | 0 | 0 | −3 | −1 | −I | 0 |
| | 1 | 0 | 0 | 0 | 0 | 0 | 0 |
| 24 | 500 | −1 | −3 | −2 | −3 | −3 | −3 |
| | 100 | 0 | −3 | −1 | −1 | −3 | −3 |
| | 50 | 0 | −3 | −2 | −3 | −3 | −3 |
| | 10 | 0 | 0 | −1 | −1 | −1 | −1 |
| | 1 | 0 | 0 | 0 | 0 | 0 | 0 |
| 25 | 500 | −3 | −3 | −2 | −3 | −3 | −3 |
| | 100 | −3 | −3 | −1 | −1 | −1 | −1 |
| | 50 | −3 | −3 | −1 | −1 | −1 | −1 |
| | 10 | −3 | −3 | 0 | 0 | −1 | −1 |
| | 1 | −1 | −1 | 0 | 0 | −1 | −1 |
| 26 | 500 | −2 | −2 | −3 | −3 | −3 | −3 |
| | 100 | 0 | 0 | −2 | −3 | −3 | −3 |
| | 50 | −3 | −3 | −2 | −3 | −3 | −3 |
| | 10 | −1 | −1 | 0 | −1 | 0 | −3 |
| | 1 | 0 | 0 | 0 | 0 | 0 | −1 |
| 27 | 500 | −2 | −3 | −3 | −3 | −3 | −3 |
| | 100 | −1 | −3 | −1 | −2 | −3 | −3 |
| | 50 | −3 | −3 | −2 | −2 | −3 | −3 |
| | 10 | 0 | 0 | 0 | 0 | 0 | −1 |
| | 1 | 0 | 0 | 0 | 0 | 0 | 0 |

Table II (continued)

| | | | | | | | |
|---|---|---|---|---|---|---|---|
| 29 | 500 | 0 | -3 | 0 | 0 | 0 | -3 |
| | 100 | 0 | -3 | 0 | 0 | 0 | -2 |
| | 50 | +1 | -1 | +1 | 0 | 0 | -2 |
| | 10 | +1 | -1 | 0 | 0 | 0 | -2 |
| | 1 | 0 | 0 | 0 | 0 | 0 | -2 |
| 30 | 500 | -3 | -3 | -3 | -3 | -3 | -3 |
| | 100 | -2 | -3 | -1 | -2 | -2 | -3 |
| | 50 | -2 | -1 | -1 | -3 | -3 | -3 |
| | 10 | 0 | 0 | 0 | 0 | 0 | -2 |
| | 1 | 0 | 0 | 0 | 0 | 0 | 0 |
| 31 | 500 | -1 | -3 | -3 | -3 | -3 | -3 |
| | 100 | 0 | -2 | -1 | -3 | -1 | -3 |
| | 50 | 0 | -1 | -1 | -3 | -2 | -3 |
| | 10 | 0 | 0 | 0 | -1 | 0 | -3 |
| | 1 | 0 | 0 | 0 | 0 | 0 | 0 |
| 32 | 500 | -3 | -3 | -3 | -3 | -3 | -3 |
| | 100 | 0 | 0 | -2 | -3 | -1 | -3 |
| | 50 | -1 | 0 | -3 | -3 | -3 | -3 |
| | 10 | 0 | 0 | -2 | -2 | -1 | -2 |
| | 1 | 0 | 0 | 0 | 0 | 0 | -1 |
| 33 | 500 | -1 | -3 | -3 | -3 | -3 | -3 |
| | 100 | 0 | -2 | -2 | -1 | -1 | -3 |
| | 50 | 0 | -1 | -2 | 0 | -1 | -2 |
| | 10 | 0 | 0 | 0 | 0 | 0 | 0 |
| | 1 | 0 | 0 | 0 | 0 | 0 | 0 |
| 34 | 500 | 0 | -3 | -1 | -3 | -3 | -3 |
| | 100 | 0 | -1 | -1 | -3 | -1 | -3 |
| | 50 | 0 | 0 | 0 | 0 | 0 | -3 |
| | 10 | 0 | 0 | 0 | 0 | 0 | 0 |
| | 1 | 0 | 0 | 0 | 0 | 0 | 0 |

Table II (continued)

| | | | | | | | |
|---|---|---|---|---|---|---|---|
| 35 | 500 | -2 | -2 | -2 | -2 | -2 | -3 |
| | 100 | 0 | -2 | -1 | -1 | -1 | -2 |
| | 50 | 0 | -1 | 0 | 0 | 0 | -1 |
| | 10 | 0 | 0 | 0 | 0 | 0 | 0 |
| | 1 | 0 | 0 | 0 | 0 | 0 | 0 |
| 36 | 500 | -2 | -3 | -2 | -3 | -3 | -3 |
| | 100 | -2 | -2 | -2 | -3 | -3 | -3 |
| | 50 | -2 | -2 | -2 | -3 | -2 | -3 |
| | 10 | 0 | -1 | -1 | -2 | -1 | -3 |
| | 1 | 0 | 0 | 0 | -1 | 0 | -1 |
| 37 | 500 | -2 | -3 | -3 | -3 | -3 | -3 |
| | 100 | -1 | -1 | -1 | -3 | -3 | -3 |
| | 50 | -1 | -1 | -1 | -2 | -3 | -2 |
| | 10 | 0 | 0 | 0 | 0 | 0 | 0 |
| | 1 | 0 | 0 | 0 | 0 | 0 | 0 |
| 38 | 500 | -2 | -3 | -2 | -2 | -3 | -3 |
| | 100 | -2 | -3 | -2 | -2 | -3 | -3 |
| | 50 | -2 | -2 | -2 | -1 | -2 | -2 |
| | 10 | -2 | -2 | -1 | 0 | -2 | -2 |
| | 1 | 0 | 0 | 0 | 0 | 0 | 0 |
| 39 | 500 | -3 | -3 | -3 | -3 | -3 | -3 |
| | 100 | -2 | -3 | -3 | -3 | -3 | -3 |
| | 50 | -1 | -2 | -3 | -3 | -3 | -3 |
| | 10 | 0 | -1 | -2 | -3 | -2 | -3 |
| | 1 | 0 | 0 | 0 | -1 | 0 | -3 |

Table II (continued)

| | | | | | | | |
|---|---|---|---|---|---|---|---|
| 40 | 500 | -1 | -3 | -3 | -3 | -3 | -3 |
| | 100 | 0 | -3 | -2 | -3 | -3 | -3 |
| | 50 | 0 | -2 | -2 | -3 | -3 | -3 |
| | 10 | 0 | 0 | 0 | -1 | 0 | -3 |
| | 1 | 0 | 0 | 0 | 0 | 0 | 0 |

1) Stem, root or leaf length measured 7 days after treatment.

2) Ratings:

-1 or +1 = less than 30% inhibition (-) or stimulation (+) of growth compared to control.

-2 or +2 = 30% to 70% inhibition or stimulation.

-3 or +3 = greater than 70% inhibition or stimulation.

Turfgrass Growth Retardation Activity

The compounds of Examples 2, 16 and 39 were further evaluated for turf growth retardant activity.

The following turf species are utilized for this evaluation: 1) Kentucky bluegrass (Poa partensis L. 'Park'); 2) red fescue (Festuca rubra L. 'Penlawn'); 3) perennial ryegrass (Lolium perenne L. 'Manhattan'); and 4) tall fescue (Festuca arundinacea Schreb. 'K31'). The seeds of these 4 turf species are planted simultaneously in 4 rows in a pan (6 5/8 inch x 5 3/8 inch). Seeding rates are as follows:

Kentucky bluegrass: 46 mg/pan
Red fescue: 91 mg/pan
Perennial ryegrass: 91 mg/pan
Tall fescue: 137 mg/pan

Seeded pans are watered with a fine nozzle until seedlings are established (usually 2 to 3 weeks after planting). After 3 to 4 weeks from date of planting, turfgrasses are mowed weekly to pan height. At 3 1/2 weeks after planting, turfgrasses are fertilized weekly with a liquid fertilizer.

After a conditioning period in a growth room, turfgrasses are treated with the test compounds as follows. Test compounds are formulated in 5 ml water + 5 ml acetone + .5 ml of 0.5 percent Triton X-155 and diluted to obtain final application rates of 8, 4, 2, 1, 0.5, 0.25, 0.125, etc., kg/hectare.

0036638

Test compounds are evaluated 2, 4 and/or 8 weeks after treatment for their turf growth retardant activity.

Turfgrass leaf growth inhibition is measured as the distance between the soil surface and the level at which the majority of erect blade tips intersect a horizontal line and is expressed in centimeters. An increase in height is calculated by subtracting 3 cm from the measured height to standardize height from the time of clipping. Root growth effects are determined on the basis of changes in root dry weight expressed in grams per pan, compared to the root dry weight of the untreated controls. Leaf color and phytotoxic effects are also observed in this test.

The compounds tested, including a commercial standard (Embark, registered trademark of 3M Company), the test species, application rates and results are summarized in Tables III-V below.

Table III

Turfgrass Retardant Activity/Phytotoxicity

% Height Inhibition (4 weeks)/Maximum phytotoxicity observed (0-5)[1] (8 weeks)

| Treatment Methods: | | Foliar Spray | | | | | Soil Drench | | |
|---|---|---|---|---|---|---|---|---|---|
| Compounds: | | A[2] | Compound of Ex. 2 | B[2] | Compound of Ex. 16 | EMBARK[3] | EMBARK | C[2] | Compound of Ex. 39 |
| Species | Rate (kg/ha) | | | | | | | | |
| Kentucky Bluegrass | 2 | 76/2 | 86/1 | 91/2 | 68/0 | 90/3 | 92/5 | 100/5 | 92/5 |
| | 1 | 62/1[4] | 86/0 | 81/1 | 46/0 | 90/2 | - | - | - |
| | 0.5 | 43/0 | 76/0 | 62/1 | 46/0 | 90/2 | 46/1 | 85/4 | 85/4 |
| | 0.25 | 24/0 | 14/0 | 19/0 | - | 29/0 | 8/0 | 81/4 | 85/1 |
| | 0.125 | - | - | - | - | - | NE[5] | 38/0 | 81/0 |
| Perennial Ryegrass | 2 | 25/3 | 75/1 | 79/3 | 68/0 | 83/3 | 83/5 | 87/5 | 87/5 |
| | 1 | 21/3 | 50/1 | 42/1 | 29/0 | 58/3 | - | - | - |
| | 0.5 | 17/0 | 29/0 | 25/0 | 36/0 | 50/3 | 10/0 | 80/4 | 83/4 |
| | 0.25 | 17/0 | 8/0 | 25/0 | - | 29/0 | NE | 73/3 | 77/1 |
| | 0.125 | - | - | - | - | - | NE | 33/0 | 50/0 |
| Creeping Red Fescue | 2 | 74/3 | 79/0 | 89/3 | 70/0 | 89/3 | 92/5 | 93/5 | 90/5 |
| | 1 | 74/2 | 79/0 | 63/1 | 57/0 | 89/2 | - | - | - |
| | 0.5 | 42/0 | 74/0 | 47/1 | 40/0 | 89/2 | 13/0 | 87/4 | 87/3 |

Table III (continued)

| | | | | | | | | | |
|---|---|---|---|---|---|---|---|---|---|
| | 0.25 | 32/0 | 16/0 | 47/0 | - | 37/0 | NE | 87/4 | 80/0 |
| | 0.125 | - | - | - | - | - | NE | 43/0 | 60/0 |
| Tall Fescue | 2 | 33/1 | 76/1 | 81/2 | 35/0 | 88/3 | 77/5 | 80/5 | 80/5 |
| | 1 | 29/0 | 43/0 | 29/2 | 21/0 | 67/3 | - | - | - |
| | 0.5 | 19/0 | 19/0 | 24/1 | 3/0 | 57/2 | NE | 77/5 | 77/4 |
| | 0.25 | 19/0 | 14/0 | 14/0 | - | 24/0 | NE | 63/4 | 67/4 |
| | 0.125 | - | - | - | - | - | NE | 33/1 | 30/0 |

1 0 = no phytotoxicity; 1 = slight leaf burn; 2 = severe tip burn; 3-5 = unacceptable.

2 A = 2-(2',5'-dimethylphenylmethylsulfinyl)pyridine N-oxide (U.S. Pat. No. 4050921, cpd. No. 50).
B = 2-(2',5'-dimethylphenylmethylsulfonyl)pyridine N-oxide (U.S. Pat. No. 4050921, cpd. No. 14).
C = 2-(1-[2',5'-dimethylphenyl]ethylsulfonyl)pyridine N-oxide (U.S. Pat. No. 4050921, cpd. No. 109).

3 N-[2,4-dimethyl-5-(trifluoromethylsulfonylaminophenyl)]acetamide.

4 Numbers in a box indicate height inhibition of 50% or more over the control with a tolerable phytotoxicity rating (1 or 0).

5 No effect.

Table IV

Effects on Leaf Color of 4 Turfgrasses

| | | Color Range (0-5)[1] | | | | | |
|---|---|---|---|---|---|---|---|
| Treatment Methods: | | Foliar Spray | | | | Soil Drench | |
| Compounds: | | A | Compound of Ex. 2 | B | Compound of Ex. 16 | C | Compound of Ex. 39 |
| Species | Rate (kg/ha) | | | | | | |
| Kentucky Bluegrass | 2 | 1-2 | 1-2 | 2 | 1-2 | 1 | 1 |
| | 1 | 1-2 | 1-2 | 1-2 | 1-2 | - | - |
| | 0.5 | 2 | 1-2 | 1-2 | 1-2 | 1-2 | 1-2 |
| | 0.25 | 2 | 2 | 1-2 | - | 1-2 | 1-2 |
| | 0.125 | - | - | - | - | 1-2 | 1-2 |
| Perennial Ryegrass | 2 | 1-2 | 1-2 | 2-3 | 1-2 | 1 | 1 |
| | 1 | 2 | 1-2 | 2 | 1-2 | - | - |
| | 0.5 | 2 | 1-2 | 2 | 1-2 | 1-2 | 1-2 |
| | 0.25 | 2 | 2 | 2 | - | 1-2 | 1-2 |
| | 0.125 | - | - | - | - | 1-2 | 1-2 |
| Creeping Red Fescue | 2 | 2 | 1-2 | 2 | 1-2 | 1 | 1 |
| | 1 | 2 | 1-2 | 1-2 | 1-2 | - | - |
| | 0.5 | 2 | 1-2 | 2 | 1-2 | 1-2 | 1-2 |

Table IV (continued)

| | | | | | | | |
|---|---|---|---|---|---|---|---|
| | 0.25 | 2 | 2 | 2 | - | 1-2 | 1-2 |
| | 0.125 | - | - | - | - | 1-2 | 1-2 |
| Tall Fescue | 2 | 2 | 2 | 2-3 | 2 | 1 | 1 |
| | 1 | 2 | 2 | 2-3 | 2 | - | - |
| | 0.5 | 2 | 2 | 2 | 2 | 1-2 | 1-2 |
| | 0.25 | 2 | 2 | 2 | - | 1-2 | 1-2 |
| | 0.125 | - | - | - | - | - | - |

0 - reddish green; 1 - dark green; 2 - green (untreated control leaf color); 3 - light green; 4 - very light green; 5 - no green color.

Table V

Effects on Combined Root Growth of 4 Turfgrass Species

| Treatment Methods: | | Foliar Spray | | | | Soil Drench | |
|---|---|---|---|---|---|---|---|
| **Compounds:** | | A | Compound of Ex. 2 | B | Compound of Ex. 16 | C | Compound of Ex. 39 |
| **Parameters** | **Rate (kg/ha)** | | | | | | |
| Root Dry Weight | 2 | 49 | 64 | 42 | 65 | - | - |
| (% of the control) | 1 | 58 | 58 | 31 | 100 | - | - |
| | 0.5 | 64 | 76 | 38 | 71 | 14 | 21 |
| | 0.25 | 60 | 81 | 69 | - | 33 | 18 |
| | 0.125 | - | - | - | - | 60 | 55 |
| Root Dry Weight/Clipping Fresh Weight | 2 | 135 | 600 | 800 | 373 | - | - |
| | 1 | 112 | 208 | 154 | 238 | - | - |
| (% of the control) | 0.5 | 77 | 165 | 112 | 150 | 1011 | 201 |
| | 0.25 | 62 | 85 | 100 | - | 1571 | 219 |
| | 0.125 | - | - | - | - | 103 | 263 |

As is evident from the data set forth above, the compounds of the present invention in comparative testing demonstrate advantageous leaf growth inhibition at relatively low application rates without adversely affecting root growth or leaf color.

While the invention has been described and illustrated with reference to certain preferred embodiments thereof, those skilled in the art will appreciate that various changes, modifications and substitutions can be made therein without departing from the spirit of the invention. For example, application rates other than those preferred ranges set forth hereinabove may be applicable due to variations in soils and planting characteristics, etc. Moreover, the specific results observed with respect to broadleaf and grass weed control as well as crop selectivity may vary depending on the specific active compounds selected and whether same are used alone or in combination with each other, i.e., mixture, or other known agents. Also, the specific formulation and the manner of applying same, e.g., soil drench, foliar spray, etc., may affect results. Accordingly, such expected changes and variations in results are contemplated in accordance with the objects and practices of the present invention. It is intended, therefore, that the invention be limited only by the scope of the claims which follow.

0036638

WHAT IS CLAIMED IS:

1. A compound of the formula:

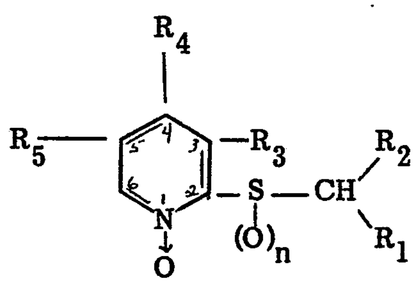

I

wherein n is 1 or 2;

$R_1$ is hydrogen or methyl;

$R_2$ is phenyl, biphenyl, naphthyl or phenyl substituted with at least one substituent selected from the group consisting of halogen, $C_1$-$C_4$ alkyl, trifluoromethyl, cyano or nitro;

$R_3$-$R_5$ are the same or different and selected from the group consisting of hydrogen, halogen, $C_1$-$C_4$ alkyl, phenyl or cyano

subject to the proviso that at least one of $R_3$-$R_5$ is other than hydrogen.

2. The compound of Claim 1 wherein said compound is selected from the group consisting of 2-(2',5'-dimethylbenzylsulfonyl)-5-bromopyridine N-oxide, 2-(2',5'-dimethylbenzylsulfonyl)-4-methylpyridine N-oxide, 2-(2',5'-dimethylbenzylsulfonyl)-4-ethylpyridine N-oxide, 2-(2',5'-dimethylbenzylsulfonyl)-4-t-butylpyridine N-oxide, 2-(2',5'-dimethylbenzylsulfonyl)-4-chloropyridine N-oxide, 2-(2'-methylbenzylsulfonyl)-4-methylpyridine N-oxide, 2-(2',6'-dichlorobenzylsulfonyl)-4-methylpyridine N-oxide, 2-(2',4',6'-trimethylbenzylsulfonyl)-4-methylpyridine N-oxide, 2-(2',6'-dichlorobenzylsulfonyl)-5-bromopyridine N-oxide, 2-(α,2',5'-trimethylbenzylsulfonyl)-4-methylpyridine N-oxide, 2-(α-methyl-2',6'-dichlorobenzylsulfonyl)-3-methylpyridine N-oxide, 2-(2',5'-dimethylbenzylsulfinyl)-4-methylpyridine N-oxide, 2-(2',5'-dimethylbenzylsulfonyl)-4,5-dimethylpyridine N-oxide, 2-(2',5'-dimethylbenzylsulfonyl)-3,4-dimethylpyridine N-oxide, 2-(4'-chlorobenzylsulfonyl)-4-methylpyridine N-oxide, 2-(4'-fluorobenzylsulfonyl)-4-methylpyridine N-oxide, 2-(3'-trifluoromethylbenzylsulfonyl)-4-methylpyridine N-oxide, 2-(2',5'-dimethylbenzylsulfinyl)-3-methylpyridine N-oxide and 2-(2',5'-dimethylbenzylsulfonyl)-3-methylpyridine N-oxide.

3. The compound of Claim 1 wherein said compound is 2-(2',5'-dimethylbenzylsulfonyl)-4-methylpyridine N-oxide.

4. A compound of the formula:

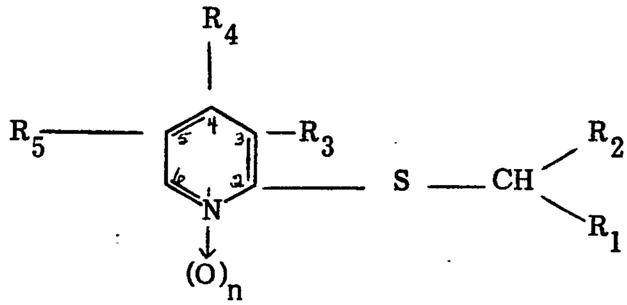

wherein n is 0 or 1;
$R_1$ is hydrogen or methyl;
$R_2$ is phenyl, biphenyl, naphthyl or phenyl substituted with at least one substituent selected from the group consisting of halogen, $C_1$-$C_4$ alkyl, trifluoromethyl, cyano or nitro;
$R_3$-$R_5$ are the same or different and selected from the group consisting of hydrogen, halogen, $C_1$-$C_4$ alkyl, phenyl or cyano
subject to the proviso that at least one of $R_3$-$R_5$ is other than hydrogen.

5. A method of regulating the growth of plants which comprises applying to the situs thereof a nonherbicidal plant growth regulant amount of a compound of the formula:

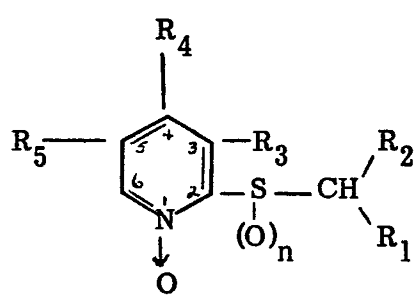

I

wherein n is 1 or 2;
$R_1$ is hydrogen or methyl;
$R_2$ is phenyl, biphenyl, naphthyl or phenyl substituted with at least one substituent selected from the group consisting of halogen, $C_1$-$C_4$ alkyl, trifluoromethyl, cyano or nitro;

$R_3$-$R_5$ are the same or different and selected from the group consisting of hydrogen, halogen, $C_1$-$C_4$ alkyl, phenyl or cyano subject to the proviso that at least one of $R_3$-$R_5$ is other than hydrogen.

6. A method for controlling undesired plants which comprises applying to the situs thereof a herbicidally effective amount of at least one active compound of the formula:

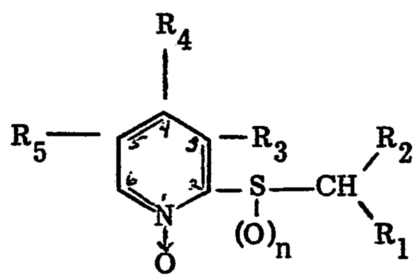

I

wherein n is 1 or 2;

$R_1$ is hydrogen or methyl;

$R_2$ is phenyl, biphenyl, naphthyl or phenyl substituted with at least one substituent selected from the group consisting of halogen, $C_1$-$C_4$ alkyl, trifluoromethyl, cyano or nitro;

$R_3$-$R_5$ are the same or different and selected from the group consisting of hydrogen, halogen, $C_1$-$C_4$ alkyl, phenyl or cyano subject to the proviso that at least one of $R_3$-$R_5$ is other than hydrogen.

7. The method of Claim 5 wherein said compound is selected from the group consisting of 2-(2',5'-dimethylbenzylsulfonyl)-5-bromopyridine N-oxide, 2-(2',5'-dimethylbenzylsulfonyl)-4-methylpyridine N-oxide, 2-(2',5'-dimethylbenzylsulfonyl)-4-ethylpyridine N-oxide, 2-(2',5'-dimethylbenzylsulfonyl)-4-t-butylpyridine N-oxide, 2-(2',5'-dimethylbenzylsulfonyl)-4-chloropyridine N-oxide, 2-(2'-methylbenzylsulfonyl)-4-methylpyridine N-oxide, 2-(2',6'-dichlorobenzylsulfonyl)-4-methylpyridine N-oxide, 2-(2',4',6'-trimethylbenzylsulfonyl)-4-methylpyridine N-oxide, 2-(2',6'-dichlorobenzylsulfonyl)-5-bromopyridine N-oxide, 2-(α,2',5'-trimethylbenzylsulfonyl)-4-methylpyridine N-oxide and 2-(α-methyl-2',6'-dichlorobenzylsulfonyl)-3-methylpyridine N-oxide.

8. The method of Claim 6 wherein said compound is selected from the group consisting of 2-(2',5'-dimethylbenzylsulfinyl)-4-methylpyridine N-oxide, 2-(2',5'-dimethylbenzylsulfonyl)-4-methylpyridine N-oxide, 2-(2',5'-dimethylbenzylsulfonyl)-5-bromopyridine N-oxide, 2-(2',5'-dimethylbenzylsulfonyl)-4-ethylpyridine N-oxide, 2-(2',5'-dimethylbenzylsulfonyl)-4-t-butylpyridine N-oxide, 2-(2',5'-dimethylbenzylsulfonyl)-4,5-dimethylpyridine N-oxide, 2-(2',5'-dimethylbenzylsulfonyl)-3,4-dimethylpyridine N-oxide, 2-(2'-methylbenzylsulfonyl)-4-methylpyridine N-oxide, 2-(4'-chlorobenzylsulfonyl)-4-methylpyridine N-oxide, 2-(4'-fluorobenzylsulfonyl)-4-methylpyridine N-oxide, 2-(3'-trifluoromethylbenzylsulfonyl)-4-methylpyridine N-oxide, 2-(2',6'-dichlorobenzylsulfonyl)-4-methylpyridine N-oxide, 2-(2',5'-dimethylbenzylsulfinyl)-3-methylpyridine N-oxide, 2-(2',5'-dimethylbenzylsulfonyl)-3-methylpyridine N-oxide, 2-(α,2',5'-trimethylbenzylsulfonyl)-4-methylpyridine N-oxide, and 2-benzylsulfonyl-4-methylpyridine N-oxide.

9. The method of Claim 5 wherein said compound is 2-(2',5'-dimethylbenzylsulfonyl)-4-methylpyridine N-oxide.

10. The method of Claims 5, 7 or 9 wherein application is by soil drench and the amount of active compound applied ranges between about 0.25 to 4.0 kg/hectare.

11. A herbicidal composition comprising a herbicidally acceptable carrier substance and a herbicidally effective amount of at least one active compound of the formula:

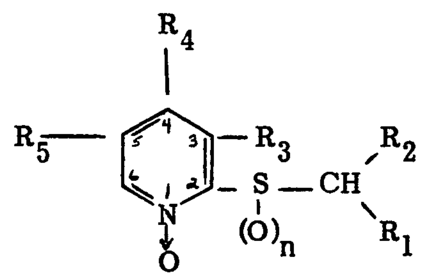

I

wherein n is 1 or 2;

$R_1$ is hydrogen or methyl;

$R_2$ is phenyl, biphenyl, naphthyl or phenyl substituted with at least one substituent selected from the group consisting of halogen, $C_1$-$C_4$ alkyl, trifluoromethyl, cyano or nitro;

$R_3$-$R_5$ are the same or different and selected from the group consisting of hydrogen, halogen, $C_1$-$C_4$ alkyl, phenyl or cyano subject to the proviso that at least one of $R_3$-$R_5$ is other than hydrogen.

12. A plant growth regulant composition comprising an effective plant growth regulant amount of at least one active compound of the formula:

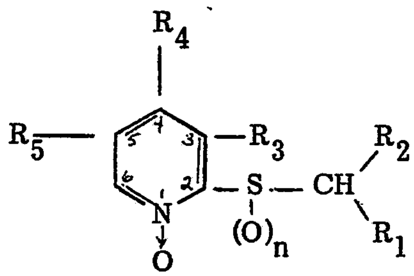

I

wherein n is 1 or 2;

$R_1$ is hydrogen or methyl;

$R_2$ is phenyl, biphenyl, naphthyl or phenyl substituted with at least one substituent selected from the group consisting of halogen, $C_1$-$C_4$ alkyl, trifluoromethyl, cyano or nitro;

$R_3$-$R_5$ are the same or different and selected from the group consisting of hydrogen, halogen, $C_1$-$C_4$ alkyl, phenyl or cyano subject to the proviso that at least one of $R_3$-$R_5$ is other than hydrogen; in combination with an agriculturally acceptable plant growth regulant carrier.